# EUROPÄISCHE PATENTSCHRIFT

(11) **EP 0 883 604 B1**
(45) Veröffentlichungstag und Bekanntmachung des Hinweises auf die Patenterteilung: **13.06.2001**
(21) Anmeldenummer: 96938106.0
(22) Anmeldetag: 06.11.1996
(51) Int. Cl.: C07C 303/24, C07C 305/06, C07C 305/10, C11D 1/14, C11D 1/16

(54) **FETTALKOHOL(ETHER)SULFATE MIT VERBESSERTEM KÄLTEVERHALTEN**
FATTY ALCOHOL (ETHER) SULPHATES WITH IMPROVED LOW-TEMPERATURE BEHAVIOUR
SULFATES (D'ETHER) D'ALCOOL GRAS A COMPORTEMENT A FROID AMELIORE

(30) Priorität: 15.11.1995 DE 19542569
(43) Veröffentlichungstag der Anmeldung: 16.12.1998
(73) Patentinhaber: Cognis Deutschland GmbH, 40589 Düsseldorf (DE)
(72) Erfinder: SCHMIDT, Wolfgang, D-40789 Monheim (DE); SCHMID, Karl-Heinz, D-40822 Mettmann (DE); NEUSS, Michael, D-50997 Köln (DE); MIDDELHAUVE, Birgit, D-40789 Monheim (DE); WUHRMANN, Juan, Carlos, D-40593 Düsseldorf (DE); ZAIKA, Dagmar, D-40822 Mettmann (DE)
(86) Internationale Anmeldenummer: EP9604838
(87) Internationale Veröffentlichungsnummer: WO9718189

(56) Entgegenhaltungen:
- WO-A-91/11506
- WO-A-91/13057
- WO-A-95/08616
- WO-A-95/11958
- JOURNAL OF THE AMERICAN OIL CHEMISTS' SOCIETY, Bd. 36, Nr. 5, Mai 1959, CHAMPAIGN, US, Seiten 208-210, XP002024049 E.E. GILBERT, ET AL.: "Sulphation with sulphur trioxide: ethenoxylated long-chain alcohols"

## Beschreibung

Die Erfindung betrifft Fettalkohol(ether)sulfate mit verbessertem Kälteverhalten, die man durch Co-Sulfatierung von Alkohol/Ethoxylat-Mischungen erhält, ein Verfahren zu ihrer Herstellung sowie die Verwendung der Ethersulfate zur Herstellung von oberflächenaktiven Mitteln.

### Stand der Technik

Fettalkoholethersulfate stellen hautverträgliche und schaumstarke Aniontenside dar, die für die Herstellung flüssiger oberflächenaktiver Mittel, wie beispielsweise manuelle Geschirrspülmittel oder Haarshampoos von besonderer Bedeutung sind. Üblicherweise kommen für diesen Einsatzzweck Produkte in Frage, die durch Sulfatierung von niedrigethoxylierten Kokosfettalkoholen hergestellt werden. Dabei werden in der Regel wäßrige Pasten hergestellt, die einen Feststoffgehalt im Bereich von 30 bis 50 Gew.-% aufweisen und bei Raumtemperatur noch fließfähig sind. Sowohl bei den Rohstoffherstellern als auch deren Kunden besteht jedoch ein Bedürfnis nach möglichst konzentrierten Produkten mit Feststoffgehalten weit oberhalb der im Markt etablierten Ethersulfate, um beispielsweise bei Lagerung und Transport Wasser einzusparen oder auf einfachem Wege Konzentrate zur Verfügung stellen zu können. Zwar ist es möglich, Ethersulfate mit einem höheren Feststoffgehalt durch entsprechende Maßnahmen bei der Neutralisation der Produkte direkt herzustellen oder wenigstens verdünnte Produkte nachträglich aufzukonzentrieren, man stellt jedoch fest, daß die bekannten niedrigethoxylierten Ethersulfate auf Basis von Kokosfettalkohol oberhalb eines Feststoffgehaltes von etwa 60 Gew.-% bei Raumtemperatur kristallisieren und dann nicht mehr pumpbar sind. Die Aufgabe der Erfindung hat somit darin bestanden, diesem Problem abzuhelfen.

### Beschreibung der Erfindung

Gegenstand der Erfindung sind Fettalkohol(ether)sulfate mit verbessertem Kälteverhalten, die man erhält, indem man Mischungen von
(a) Fettalkoholen der Formel **(I),**

   **R**^{**1**}**OH (I)**

   in der R¹ für lineare und/oder verzweigte, aliphatische Alkylreste mit 8 bis 22, vorzugsweise 12 bis 18 Kohlenstoffatomen steht, und
(b) Fettalkoholethoxylaten der Formel **(II),**

   **R**^{**2**}**O(CH**_{**2**}**CH**_{**2**}**O)**_{**n**}**H (II)**

   in der R² für lineare und/oder verzweigte, aliphatische Alkylreste mit 8 bis 22, vorzugsweise 12 bis 18 Kohlenstoffatomen und n für Zahlen von 1,9 bis 3,9 steht,
im Gewichtsverhältnis 20 : 80 bis 80 : 20, vorzugsweise 20 : 60 bis 60 : 40 und insbesondere 30 : 70 bis 35 : 65, in an sich bekannter Weise cosulfatiert und anschließend mit wäßrigen Basen neutralisiert.

Überraschenderweise wurde gefunden, daß die Fettalkohol(ether)sulfate selbst dann noch bei 20°C fließfähig und pumpbar sind, wenn ihr Feststoffgehalt deutlich oberhalb von 60 Gew.-% liegt. Demgegenüber liegen Ethersulfate vergleichbarer Kettenlänge und Ethoxylierungsgrades, die man durch alleinige Sulfatierung eines entsprechenden Ethoxylats hergestellt hat, unter den gleichen Bedingungen als schnittfeste, kristallisierte Pasten vor.

Ein weiterer Gegenstand der Erfindung betrifft ein Verfahren zur Herstellung von Fettalkohol(ether)sulfaten mit verbessertem Kälteverhalten, bei dem man Mischungen von
(a) Fettalkoholen der Formel (I),

   **R**^{**1**}**OH (I)**

   in der R¹ für lineare und/oder verzweigte, aliphatische Alkylreste mit 8 bis 22, vorzugsweise 12 bis 18 Kohlenstoffatomen steht, und
(b) Fettalkoholethoxylaten der Formel **(II),**

   **R**^{**2**}**O(CH**_{**2**}**CH**_{**2**}**O)**_{**n**}**H (II)**

   in der R² für lineare und/oder verzweigte, aliphatische Alkylreste mit 8 bis 22, vorzugsweise 12 bis 18 Kohlenstoffatomen und n für Zahlen von 1,9 bis 3,9 steht,
im Gewichtsverhältnis 20 : 80 bis 80 : 20, vorzugsweise 20 : 60 bis 40 : 80 und insbesondere 30 : 70 bis 35 : 65 in an sich bekannter Weise cosulfatiert und anschließend mit wäßrigen Basen neutralisiert.

### Fettalkohole

Typische Beispiele für Fettalkohole, die als Komponente (a) in Betracht kommen, sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen wie beispielsweise Kokos-, Palm-, Palmkern- oder Talgfettalkohol.

Unter den verzweigten Einsatzstoffen sind beispielsweise Alkohole bevorzugt, die unter den Handelsbezeichnungen Dobanol® und Lial® im Handel sind.

### Fettalkoholethoxylate

Als Fettalkoholethoxylate, die die Komponente (b) ausmachen, kommen Anlagerungsprodukte von 1,9 bis 3,9 und vorzugsweise 2 bis 3 Mol Ethylenoxid an die oben genannten Fettalkohole in Betracht. Bevorzugt ist der Einsatz von Fettalkoholethoxylaten mit 12 bis 18 Kohlenstoffatomen im Fettalkylrest, wobei es sich als besonders vorteilhaft erwiesen hat, wenn die Kohlenstoffketten der Fettalkohole der Komponente (a) und der Ethoxylate der Komponente (b) identisch sind. Die Ethoxylate können eine konventionelle oder auch eingeengte Homologenverteilung aufweisen.

### Sulfatierung

Die Sulfatierung der Alkohol/Ethoxylat-Mischungen kann in der für Fettsäureniedrigalkylester bekannten Weise **[J.Falbe (ed.), "Surfactants in consumer products", Springer Verlag, Berlin-Heidelberg, 1987, S. 61]** erfolgen, wobei Reaktoren, die nach dem Fallfilmprinzip arbeiten, bevorzugt sind. Als Sulfiermittel kommen Chlorsulfonsäure und insbesondere gasförmiges Schwefeltrioxid in Betracht. Letzteres wird üblicherweise mit einem inerten Gas, vorzugsweise Luft oder Stickstoff verdünnt und in Form eines Gasgemisches, welches das Sulfieragens in einer Konzentration von 1 bis 8, insbesondere 2 bis 5 Vol.-% enthält, eingesetzt.

Das molare Einsatzverhältnis von Alkohol/Ethoxylat-Mischung zu Sulfatierungsmittel kann 1 : 0,95 bis 1 : 1,3 und vorzugsweise 1 : 1 bis 1 : 1,05 betragen. Üblicherweise wird die Sulfatierung bei Temperaturen im Bereich von 25 bis 70 durchgeführt. Im Hinblick auf die Viskosität der Einsatzstoffe einerseits und die Farbqualität der resultierenden Sulfatierungsprodukte andererseits, hat es sich als optimal erwiesen, die Reaktion in einem Temperaturbereich von 30 bis 55°C durchzuführen.

### Neutralisation

Die bei der Sulfatierung anfallenden sauren Sulfatierprodukte werden in wäßrige Basen eingerührt, neutralisiert und auf einen pH-Wert von 6,5 bis 8,5 eingestellt. Als Basen für die **Neutralisation** kommen Alkalimetallhydroxide wie Natrium-, Kalium- und Lithiumhydroxid, Erdalkalimetalloxide und -hydroxide wie Magnesiumoxid, Magnesiumhydroxid, Calciumoxid und Calciumhydroxid, Ammoniak, Mono-, Di- und Tri-C₂₋₄-Alkanolamine, beispielsweise Mono-, Di- und Triethanolamin sowie primäre, sekundäre oder tertiäre C₁₋₄-Alkylamine sowie Glucamine in Betracht. Die Neutralisationsbasen gelangen dabei vorzugsweise in Form 5 bis 55 Gew.-%iger wäßriger Lösungen zum Einsatz, wobei 25 bis 50 Gew.-%ige wäßrige Natriumhydroxidlösungen bevorzugt sind.

In einer bevorzugten Ausführungsform der Erfindung wird die Neutralisation so ausgeführt, daß Pasten mit einem Feststoffgehalt im Bereich von 60 bis 80 und insbesondere 65 bis 75 Gew.-% entstehen.

### Bleiche und Konservierung

Die Sulfatierungsprodukte können nach Neutralisation in an sich bekannter Weise durch Zusatz von Wasserstoffperoxid- oder Natriumhypochloritlösung gebleicht werden. Dabei werden, bezogen auf den Feststoffgehalt in der Lösung der Sulfatierungsprodukte, 0,2 bis 2 Gew.-% Wasserstoffperoxid, berechnet als 100 %ige Substanz, oder entsprechende Mengen Natriumhypochlorit eingesetzt. Der pH-Wert der Lösungen kann unter Verwendung geeigneter Puffermittel, z. B. mit Natriumphosphat oder Citronensäure konstant gehalten werden. Zur Stabilisierung gegen Bakterienbefall empfiehlt sich ferner eine Konservierung, z. B. mit Formaldehydlösung, p-Hydroxybenzoat, Sorbinsäure oder anderen bekannten Konservierungsstoffen.

### Gewerbliche Anwendbarkeit

Die erfindungsgemäßen Fettalkohol(ether)sulfate weisen ein vorteilhaftes Kälteverhalten auf, d.h. sind auch bei Feststoffgehalten oberhalb von 65 Gew.-% noch fließ- und pumpfähig. Sie eignen sich daher als Rohstoffe zur Herstellung zahlreicher oberflächenaktiver Mittel wie beispielsweise Flüssigwaschmittel, Geschirrspülmittel oder Haarshampoos, in denen sie in Mengen im Bereich von 1 bis 50 und vorzugsweise 5 bis 25 Gew.-% .- bezogen auf die Mittel - enthalten sein können.

### Beispiele

### Beispiel 1

In einem 1-1-Sulfierreaktor mit Gaseinleitungsrohr und Mantelkühlung wurde eine Mischung aus 70 g (0,35 Mol) technischer C_{12/14}-Kokosfettalkohol (Lorol® Spezial, Henkel KGaA, Düsseldorf/FRG) und 187 g (0,65 Mol) C_{12/14}-Kokosfettalkohol+2EO (Dehydol® LS2, Henkel KGaA) vorgelegt und auf 45°C erhitzt. In die Mischung wurden innerhalb von 20 min 84 g (1,05 Mol) gasförmiges Schwefeltrioxid (3 Vol-%ig in Stickstoff) eingeleitet, das zuvor aus einer entsprechenden Menge Oleum ausgetrieben worden war. Das rohe Sulfatierungsprodukt wurde zusammen mit 50 Gew.-%iger Natriumhydroxidlösung neutralisiert. Das resultierende Gemisch der Alkylsulfat- und Ethersulfat-Natriumsalze wies einen Feststoffgehalt von 70 Gew.-% auf; der mittlere Ethoxylierungsgrad der Mischung betrug 1,2. Das Produkt zeigte bei 20°C bzw. 25°C Viskositäten von 14.000 bzw. 10.000 mPas (Brook-field, RVF-Viskosimeter, 20 Upm, Spindel 1), war fließfähig und pumpbar.

### Beispiele 2 und 3

Beispiel 1 wurde unter Einsatz von a) 60 g (0,3 Mol) Kokosfettalkohol und 202 g (0,7 Mol) Kokosfettalkohol+2EO bzw. b) 80 g (0,4 Mol) Kokosfettalkohol und 173 g (0,6 Mol) Kokosfettalkohol+2EO wiederholt. Bei Feststoffgehalten von wiederum 70 Gew.-% wurden bei 20°C in beiden Fällen fließfähige und pumpbare Pasten erhalten.

### Vergleichsbeispiele V1 und V2

Analog Beispiel 1 wurden a) 1 Mol eines C_{12/14}-Kokosfettalkohol+IEO-Adduktes bzw. b) 1 Mol eines C_{12/14}-Kokosfettalkohol+1,2EO-Adduktes mit 1,05 Mol Schwefeltrioxid sulfatiert und anschließend mit Natriumhydroxidlösung neutralisiert. Die Produkte wurden jeweils auf einen Feststoffgehalt von 70 Gew.-% eingestellt. Beide Produkte waren jedoch sowohl bei 20 als auch bei 25°C fest.

## Patentansprüche

1. Fettalkohol(ether)sulfate mit verbessertem Kälteverhalten, dadurch erhältlich, daß man Mischungen von
(a) Fettalkoholen der Formel **(I),**
**R**^{**1**}**OH (I)**
in der R¹ für lineare und/oder verzweigte, aliphatische Alkylreste mit 8 bis 22 Kohlenstoffatomen steht, und
(b) Fettalkoholethoxylaten der Formel **(II),**
**R**^{**2**}**O(CH**_{**2**}**CH**_{**2**}**O)**_{**n**}**H (II)**
in der R² für lineare und/oder verzweigte, aliphatische Alkylreste mit 8 bis 22 Kohlenstoffatomen und n für Zahlen von 1,9 bis 3,9 steht,
im Gewichtsverhältnis 20 : 80 bis 80 : 20 in an sich bekannter Weise cosulfatiert und anschließend mit wäßrigen Basen neutralisiert.

2. Verfahren zur Herstellung von Fettalkohol(ether)sulfaten mit verbessertem Kälteverhalten, bei dem man Mischungen von
(a) Fettalkoholen der Formel **(I),**
**R**^{**1**}**OH (I)**
in der R¹ für lineare und/oder verzweigte, aliphatische Alkylreste mit 8 bis 22 Kohlenstoffatomen steht, und
(b) Fettalkoholethoxylaten der Formel (II),
**R**^{**2**}**O(CH**_{**2**}**CH**_{**2**}**O)**_{**n**}**H (II)**
in der R² für lineare und/oder verzweigte, aliphatische Alkylreste mit 8 bis 22 Kohlenstoffatomen und n für Zahlen von 1,9 bis 3,9 steht,
im Gewichtsverhältnis 20 : 80 bis 80 : 20 in an sich bekannter Weise cosulfatiert und anschließend mit wäßrigen Basen neutralisiert.

3. Verfahren nach Anspruch 2, **dadurch gekennzeichnet,** daß man als Sulfatiermittel Chlorsulfonsäure oder gasförmiges Schwefeltrioxid einsetzt.

4. Verfahren nach den Ansprüchen 2 und 3, **dadurch gekennzeichnet,** daß man die Sulfatierung in einem kontinuierlichen Reaktor durchführt, der nach dem Fallfilmprinzip arbeitet.

5. Verfahren nach den Ansprüchen 2 bis 4, **dadurch gekennzeichnet,** daß man die Alkohol/Ethoxylat-Mischungen und die Sulfatiermittel im molaren Verhältnis von 1 : 0,95 bis 1 : 1,3 einsetzt.

6. Verfahren nach den Ansprüchen 2 bis 5, **dadurch gekennzeichnet,** daß man die Sulfatierung bei Temperaturen im Bereich von 25 bis 70°C durchführt.

7. Verfahren nach den Ansprüchen 2 bis 6, **dadurch gekennzeichnet,** daß man die Neutralisation mit 5 bis 55 Gew.-%igen wäßrigen Basen aus der von Alkalimetallhydroxiden, Erdalkalimetalloxiden und -hydroxiden, Ammoniak, Mono-, Di- und Tri-C₂₋₄-Alkanolaminen sowie primären, sekundären und tertiären C₁₋₄-Alkylaminen sowie Glucaminen gebildeten Gruppe durchführt.

8. Verfahren nach den Ansprüchen 2 bis 7, **dadurch gekennzeichnet,** daß man einen Feststoffgehalt im Bereich von 60 bis 80 Gew.-% einstellt.

9. Verwendung von Fettalkohol(ether)sulfaten nach Anspruch 1 zur Herstellung von oberflächenaktiven Mitteln.

## Claims

1. Fatty alcohol (ether) sulfates with improved low-temperature behavior which are obtainable by co-sulfating mixtures of
(a) fatty alcohols corresponding to formula **(I):**
**R**^{**1**}**OH (I)**
in which R¹ represents linear and/or branched aliphatic alkyl groups containing 8 to 22 carbon atoms,
and
(b) fatty alcohol ethoxylates corresponding to formula **(II):**
**R**^{**2**}**O(CH**_{**2**}**CH**_{**2**}**O)**_{**n**}**H (II)**
in which R² represents linear and/or branched aliphatic alkyl groups containing 8 to 22 carbon atoms and n is a number of 1.9 to 3.9,
in a ratio by weight of 20:80 to 80:20 by methods known per se and neutralizing the mixtures thus co-sulfated with aqueous bases.

2. A process for the production of fatty alcohol (ether) sulfates with improved low-temperature behavior, in which mixtures of
(a) fatty alcohols corresponding to formula **(I):**
**R**^{**1**}**OH (I)**
in which R¹ represents linear and/or branched aliphatic alkyl groups containing 8 to 22 carbon atoms,
and
(b) fatty alcohol ethoxylates corresponding to formula **(II):**
**R**^{**2**}**O(CH**_{**2**}**CH**_{**2**}**O)**_{**n**}**H (II)**
in which R² represents linear and/or branched aliphatic alkyl groups containing 8 to 22 carbon atoms and n is a number of 1.9 to 3.9,
in a ratio by weight of 20:80 to 80:20 are co-sulfated by methods known per and then neutralized with aqueous bases.

3. A process as claimed in claim 2, characterized in that chlorosulfonic acid or gaseous sulfur trioxide is used as the sulfating agent.

4. A process as claimed in claims 2 and 3, characterized in that the sulfation reaction is carried out in a continuous reactor operating on the falling-film principle.

5. A process as claimed in claims 2 to 4, characterized in that the alcohol/ethoxylate mixtures and the sulfating agent are used in a molar ratio of 1:0.95 to 1:1.3.

6. A process as claimed in claims 2 to 5, characterized in that the sulfation reaction is carried out at temperatures of 25 to 70°C.

7. A process as claimed in claims 2 to 6, characterized in that the neutralization step is carried out with 5 to 55% by weight aqueous bases from the group consisting of alkali metal hydroxides, alkaline earth metal oxides and hydroxides, ammonia, mono-, di- and tri-C₂₋₄-alkanolamines and primary, secondary and tertiary C₁₋₄ alkylamines and also glucamines.

8. A process as claimed in claims 2 to 7, characterized in that a solids content of 60 to 80% by weight is adjusted.

9. The use of the fatty alcohol (ether) sulfates claimed in claim 1 for the production of surface-active compositions.

## Revendications

1. (Ether)sulfates d'alcool gras à comportement à froid amélioré, que l'on obtient en cosulfatant de façon connue, puis en neutralisant avec des bases aqueuses des mélanges :
(a) d'alcools gras de formule (I),
**R**^{**1**}**OH (I)**
dans laquelle R¹ représente des radicaux alkyle aliphatiques linéaires et/ou ramifiés comprenant de 8 à 22 atomes de carbone, et
(b) d'éthoxylates d'alcools gras de formule (II)
**R**^{**2**}**O(CH**_{**2**}**CH**_{**2**}**O)**_{**n**}**H (II)**
dans laquelle R² représente des radicaux alkyle aliphatiques linéaires ou ramifiés comportant de 8 à 22, et n représente des nombres allant de 1,9 à 3,9,
dans un rapport pondéral de 20:80 à 80:20.

2. Procédé de fabrication d'(éther)sulfates d'alcools gras ayant un comportement à froid amélioré, dans lequel on cosulfate de façon connue, puis on neutralise avec des bases aqueuses des mélanges
(a) d'alcools gras de formule (I),
**R**^{**1**}**OH (I)**
dans laquelle R¹ représente des radicaux alkyle aliphatiques et/ou linéaires ramifiés comportant de 8 à 22 atomes de carbone, et
(b) d'éthoxylates d'alcools gras de formule (II),
**R**^{**2**}**O(CH**_{**2**}**CH**_{**2**}**O)**_{**n**}**H (II)**
dans laquelle R² représente des radicaux alkyle aliphatiques linéaires et/ou ramifiés comportant de 8 à 22 atomes de carbone, et n représente des nombres allant de 1,9 à 3,9,
dans un rapport pondéral de 20:80 à 80:20.

3. Procédé selon la revendication 2,
caractérisé en ce qu'
on utilise comme agent de sulfatation l'acide chlorosulfonique ou l'anhydride sulfurique gazeux.

4. Procédé selon les revendications 2 et 3,
caractérisé en ce qu'
on réalise la sulfatation dans un réacteur en continu qui fonctionne selon le principe de la pellicule tombante.

5. Procédé selon les revendications 2 à 4,
caractérisé en ce qu'
on utilise le mélange alcool/éthoxylate et les produits de sulfatation dans un rapport molaire de 1:0,95 à 1:1,3.

6. Procédé selon les revendications 2 à 5,
caractérisé en ce qu'
on réalise la sulfatation à des températures comprises entre 25 et 70°C.

7. Procédé selon les revendications 2 à 6,
caractérisé en ce qu'
on conduit la neutralisation avec des bases aqueuses à 5 à 55 % en poids, du groupe formé par les hydroxydes de métaux alcalins, les oxydes et hydroxydes de métaux alcalino-terreux, l'ammoniac, les mono-, di- et trialcanol en C₂ à C4-amines ainsi que les alkyle en C₁ à C₄-amines, primaires, secondaires et tertiaires, et également les glucamines.

8. Procédé selon les revendications 2 à 7,
caractérisé en ce qu'
on établit une teneur en solides comprise entre 60 et 80 % en poids.

9. Utilisation d'(éther)sulfates d'alcools gras selon la revendication 1 pour la production d'agents tensioactifs.
